# EUROPEAN PATENT APPLICATION

(11) **EP 3 666 270 A1**
(43) Date of publication of application: **17.06.2020**
(21) Application number: 18844283.4
(22) Date of filing: 07.08.2018
(51) Int. Cl.: A61K 31/4152, A61K 9/08, A61K 47/18, A61P 21/00, A61P 25/00

(54) **DRUG CONTAINING PYRAZOLONE DERIVATIVE**

(30) Priority: 08.08.2017 JP 2017152941; 27.09.2017 JP 2017185641
(71) Applicant: Nobelpharma Co., Ltd., Chuo-ku Tokyo 1040033 (JP)
(72) Inventor: TANAKA, Masahiko, Tokyo 156-0052 (JP); YAMAMOTO, Yorihiro, Tokyo 167-0043 (JP)
(74) Representative: Schön, Christoph
(86) International application number: PCT/JP2018/029581
(87) International publication number: WO 2019/031495

(57) **Abstract**

An object of the present invention is to provide a highly stable medicament comprising a pyrazolone derivative such as edaravone, which is free of sodium bisulfite. The present invention provides a medicament, comprising
(a) a pyrazolone derivative such as 3-methyl-1-phenyl-2-pyrazolin-5-one;
(b) a glutathione analogue, and
(c) an aqueous solvent, which comprises none of sulfite, bisulfite, and pyrosulfite, and is subjected to deoxygenation treatment.

## Description

### Technical Field

The present invention relates to a medicament comprising a pyrazolone derivative, a glutathione analogue, and an aqueous solvent.

### Background Art

3-Methyl-1-phenyl-2-pyrazolin-5-one is also referred to as "5-methyl-2-phenyl-2,4-dihydro-3H-pyrazole-3-one," and its International Nonproprietary Name (INN) is "edaravone" and the name according to Japanese Accepted Names for Pharmaceuticals (JAN) is *edaravone* (in Japanese) or edaravone.

Edaravone was approved as a prescription drug in Japan in 2001 with the indications "Improvement of neurological symptoms, disability in activities of daily living, and functional impairment associated with acute ischemic stroke" and the dose and administration "The usual adult dosage is 30 mg of edaravone administered twice daily by intravenous infusion over 30 minutes in the morning and the evening. Treatment with edaravone should be initiated within 24 hours after the onset of the disease and can be continued for up to 14 days." (Japanese Pharmacopoeia: package insert of the edaravone injection, RADICUT (registered trademark) Injection 30mg, April 2001 (International Birth Day (IBD)), initially marketed in June 2001, Approval No. 21300AMZ00377, revised in June 2015 (18th edition); and package insert of the edaravone injection RADICUT (registered trademark) BAG for I.V Infusion 30mg, initially marketed in May 2010, Approval No. 22200AMX00224, revised in June 2015 (9th edition)).

Subsequently, the following additional indications and dose and administration of edaravone were approved in 2015 in Japan: Indications: "Slowing of progression of functional impairment in patients with amyotrophic lateral sclerosis (ALS);" Dose and administration: "The usual adult dosage is 60 mg of edaravone administered once daily by intravenous infusion over 60 minutes. Usually, edaravone should be administered in 28-day cycles, each consisting of a treatment period and a rest period. In the first cycle, edaravone should be administered for 14 consecutive days, followed by a 14-day rest period. In the second and subsequent cycles, a total of 10 doses of once-daily edaravone should be administered during a 14-day treatment period, followed by a 14-day rest period."

There are original products of the edaravone injection with indications for both acute ischemic stroke and amyotrophic lateral sclerosis (ALS), which are RADICUT Injection 30mg (20 mL per ampoule) and RADICUT BAG for I.V Infusion 30mg (100 mL per bag). The added amounts of sodium bisulfite and L-cysteine hydrochloride hydrate, which will be described later, are the same as 20 mg and 10 mg.

Further, the U.S. Food and Drug Administration (FDA) approved edaravone for treatment of patients with ALS (amyotrophic lateral sclerosis) in 2017. The statement made when FDA announced the approval for ALS describes that sodium bisulfite may cause anaphylactic symptoms and life-threatening episodes in susceptible people"(https://www.fda.gov/NewsEvents/Newsroom/PressAnnouncements/ucm557102 .htm; and https://www.accessdata.fda.gov/drugsatfda_docs/label/2017/2091761bl.pdf).

Edaravone is the world's first brain protective agent (free radical scavenger), and is the first medicinal product which was approved for the indication for "improvement of functional impairment" associated with acute ischemic stroke. In addition, ALS is a very severe intractable neurological disease with unknown cause, and the effects of Riluzole that is an existing glutamate release inhibitor are limited, and an effective treatment method has been demanded. Edaravone provides a new treatment option for ALS, and was approved because it was determined that it is meaningful to provide edaravone to the medical field. Edaravone is a compound that is expected to have clinical usefulness as a therapeutic agent for acute cerebral infarction and ALS, which has not been always satisfactory so far.

In Patent Document 1, since 3-methyl-1-phenyl-2-pyrazolin-5-one (edaravone) undergoes oxidation in an aqueous solution, the addition of sulfite as an antioxidant was examined during the design of an injection, and the addition of an antioxidant was found to increase stability. However, the effects thereof were insufficient. Further, other additives were examined. Although a cysteine alone did not show such stabilizing effects, stability was improved by combining sulfite and a cysteine. Therefore, sodium bisulfite and L-cysteine hydrochloride have been added.

In the stability test of the formulation, potentially carcinogenic phenylhydrazine was present in a small amount. However, as the amount was lower than the allowable exposure limit set by the American Conference of Governmental Industrial Hygienists (ACGIH), which is the strictest exposure limit for phenylhydrazine, it was judged that the formulation does not increase the risk of carcinogenesis associated with phenylhydrazine intake (Non-Patent Document 1).

In addition, along with the launch of generic drugs in recent years, a comparison of radical scavenging ability with the original product (the above formulation) has been made, and reports mentioning differences in additives have been made (Non-Patent Document 2 and 3).

### Prior Art Documents

### Patent Documents

Patent Document 1: JP 7-121861 B (1995)

### Non-Patent Documents

Non-Patent Document 1: Shinyaku Shonin Joho Shu (new drug approval information reports): Edaravone [RADICUT Injection 30mg], 2001; No.11.
Non-Patent Document 2: Keishi Yamasaki, Takako Ishiguro, Hakaru Seo: Comparison of radical scavenging activity of original and generic drugs of edaravone injections, Journal of New Remedies & Clinics, 2011; 60; 2413-2419.
Non-Patent Document 3: Kenji Yoshida, Masakazu Kobayashi, Hideo Saito, Taro Suzuki, Koji Yoshida, Kuniaki Ogasawara: Comparison of radical scavenging ability by spectrophotometry (2,2-diphenyl-1-picrylhydrazyl) of original and generic drugs of edaravone formulations, Japanese Journal of Stroke, 2013; 35: 375-377.

### Summary of Invention

### Object to be Solved by the Invention

Potentially carcinogenic phenylhydrazine was present in a small amount in the formulation of Patent Document 1. Edaravone will be administered repeatedly for longer periods of time because of the approval for amyotrophic lateral sclerosis (ALS). Therefore, the development of stable injections without generation of phenylhydrazine is desired. In addition, along with the launch of generic drugs, it has been reported that differences in additives used for improving stability affect radical scavenging ability, which is the basis of efficacy. There is a demand to develop better pharmaceutical compositions that can reliably maintain the stability of edaravone. Further, the development of a medicament comprising edaravone free of sodium bisulfite which may cause anaphylactic symptoms noted by the U.S. Food and Drug Administration (FDA) is desired.

An object of the present invention is to provide a highly stable medicament comprising a pyrazolone derivative such as edaravone, which is free of sodium bisulfite.

### Means for Solving the Object

The present inventors made intensive studies in order to achieve the above object. As a result, the present inventors found that a deoxygenated medicament comprising edaravone, a glutathione analogue, and an aqueous solvent can be maintained stably without using sodium bisulfite, which may cause anaphylactic symptoms. This has led to the completion of the present invention.

Specifically, the following inventions are provided according to the present invention.
[1] A medicament, comprising:
   (a) a compound represented by the following formula (I) or a physiologically acceptable salt thereof, or a hydrate or solvate thereof;
   (b) a glutathione analogue, and
   (c) an aqueous solvent,
   which comprises none of sulfite, bisulfite, and pyrosulfite, and is subjected to deoxygenation treatment: 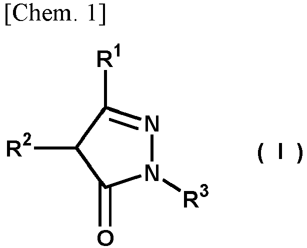 wherein R¹ represents a hydrogen atom, aryl, C₁-C₅ alkyl, or alkoxycarbonylalkyl having a total carbon number of 3 to 6, and R² represents a hydrogen atom, aryloxy, arylmercapto, C₁-C₅ alkyl, or C₁-C₃ hydroxyalkyl; or R¹ and R² together represent C₃-C₅ alkylene, and R³ represents a hydrogen atom, C₁-C₅ alkyl, C₅-C₇ cycloalkyl, C₁-C₃ hydroxyalkyl, benzyl, naphthyl, or phenyl, or phenyl substituted with 1 to 3 identical or different substituents selected from the group consisting of C₁-C₅ alkoxy, C₁-C₃ hydroxyalkyl, alkoxycarbonyl having a total carbon number of 2 to 5, C₁-C₃ alkylmercapto, C₁-C₄ alkylamino, dialkylamino having a total carbon number of 2 to 8, a halogen atom, trifluoromethyl, carboxyl, cyano, a hydroxy group, nitro, amino, and acetamide.
[2] The medicament according to the above [1], wherein the compound represented by the formula (1) is 3-methyl-1-phenyl-2-pyrazolin-5-one.
[3] The medicament according to the above [1] or [2] wherein the glutathione analogue is glutathione.
[4] The medicament according to any one of the above [1] to [3], wherein the aqueous solvent is water.
[5] The medicament according to any one of the above [1] to [4], wherein the deoxygenation treatment is nitrogen substitution treatment.
[6] The medicament according to any one of the above [1] to [5], which is an injection.
[7] The medicament according to any one of the above [1] to [6], wherein the content of the compound represented by the formula (I) or a physiologically acceptable salt thereof, or a hydrate or solvate thereof is 15 mg to 3000 mg, and the liquid volume of the medicament is 10 mL to 2000 mL.
[8] The medicament according to any one of the above [1] to [7], wherein the content of the compound represented by the formula (I) or a physiologically acceptable salt thereof, or a hydrate or solvate thereof is 30 mg, and the liquid volume of the medicament is 15 mL to 200 mL.
[9] The medicament according to any one of the above [1] to [7], wherein the content of the compound represented by the formula (I) or a physiologically acceptable salt thereof, or a hydrate or solvate thereof is 60 mg, and the liquid volume of the medicament is 30 mL to 200 mL.
[10] The medicament according to any one of the above [1] to [7], wherein the content of the compound represented by the formula (I) or a physiologically acceptable salt thereof, or a hydrate or solvate thereof in the liquid volume of the medicament is 30 mg/20 mL, 30 mg/100 mL, 60 mg/200 mL, 150 mg/100 mL, 300 mg/200 mL, 600 mg/400 mL, 1800 mg/1200 mL, or 2250 mg/1500 mL.

### Advantageous Effects of Invention

The medicament of the present invention is an aqueous solution of a pyrazoline derivative comprising a glutathione analogue. The medicament of the present invention is excellent in long-term stability, and substantially no phenylhydrazine, an impurity that has been observed in a small amount in conventional injections, is generated therein.

### Brief Description of Drawings

[Fig. 1] Fig. 1 shows the edaravone concentrations of the samples obtained in Example 1 and Comparative Examples 1, 2, and 3.
[Fig. 2] Fig. 2 is a view of pictures of the samples obtained in Example 1 and Comparative Examples 1, 2, and 3 showing the appearance of each sample (liquid volume of 20 mL).
[Fig. 3] Figure 3 shows the results of confirming the presence or absence of phenylhydrazine (60°C, 4 weeks later, liquid volume of 20 mL) in the samples obtained in Example 1 and Comparative Examples 1, 2, and 3.
[Fig. 4] Fig. 4 shows the edaravone concentrations of the samples obtained in Example 2 and Comparative Examples 4, 5, and 6.
[Fig. 5] Fig. 5 is a view of pictures of the samples obtained in Example 2 and Comparative Examples 4, 5, and 6 showing the appearance of each sample (liquid volume of 100 mL).
[Fig. 6] Fig. 6 shows the edaravone concentrations of the samples obtained in Example 3 and Comparative Examples 7 and 8.
[Fig. 7] Fig. 7 is a view of pictures of the samples obtained in Example 3 and Comparative Examples 7 and 8 showing the appearance of each sample (liquid volume of 10 mL).
[Fig. 8] Fig. 8 shows the edaravone concentrations of the samples obtained in Examples 4, 5, 6, 7, and 8 and Comparative Example 9. In addition, the presence or absence of insoluble foreign matter is indicated.

### Embodiment of Carrying out the Invention

Hereinafter, embodiments of the present invention will be described. The medicament of the present invention comprises:
(a) a compound represented by the following formula (I) or a physiologically acceptable salt thereof, or a hydrate or solvate thereof;
(b) a glutathione, and
(c) an aqueous solvent,
   and is characterized in that it comprises none of sulfite, bisulfite, and pyrosulfite, and is subjected to deoxygenation treatment. The medicament of the present invention is also a combined medicament which is a combination of: (a) a compound represented by the following formula (I) or a physiologically acceptable salt thereof, or a hydrate or solvate thereof; and (b) a glutathione: 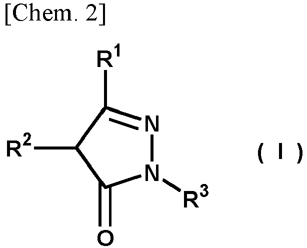 wherein R¹ represents a hydrogen atom, aryl, C₁-C₅ alkyl, or alkoxycarbonylalkyl having a total carbon number of 3 to 6, and R² represents a hydrogen atom, aryloxy, arylmercapto, C₁-C₅ alkyl, or C₁-C₃ hydroxyalkyl; or R¹ and R² together represent C₃-C₅ alkylene, and R³ represents a hydrogen atom, C₁-C₅ alkyl, C₅-C₇ cycloalkyl, C₁-C₃ hydroxyalkyl, benzyl, naphthyl, or phenyl, or phenyl substituted with 1 to 3 identical or different substituents selected from the group consisting of C₁-C₅ alkoxy, C₁-C₃ hydroxyalkyl, alkoxycarbonyl having a total carbon number of 2 to 5, C₁-C₃ alkylmercapto, C₁-C₄ alkylamino, dialkylamino having a total carbon number of 2 to 8, a halogen atom, trifluoromethyl, carboxyl, cyano, a hydroxy group, nitro, amino, and acetamide.

The medicament of the present invention comprises a pyrazolone derivative represented by the formula (I) or a physiologically acceptable salt thereof, or a hydrate or solvate thereof as an active ingredient.

The compound represented by the formula (I) may have a structure represented by the following formula (I') or (I ") due to tautomerism. In the formula (I) described herein, one tautomer is shown for convenience, but the existence of the following tautomers is obvious to a person skilled in the art. As an active ingredient of the medicament of the present invention, a compound represented by the following formula (I') or (I") or a physiologically acceptable salt thereof, or a hydrate or solvate thereof may be used.

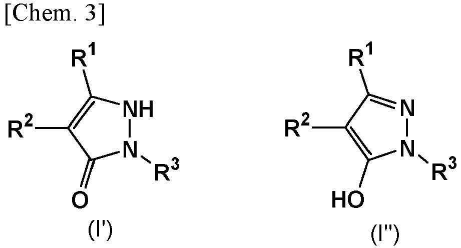

In the formula (I), the aryl group in the definition of R¹ may be either a monocyclic or polycyclic aryl group. Examples thereof include not only a phenyl group and a naphthyl group but also an alkyl group such as a methyl group or butyl group, an alkoxy group such as a methoxy group or butoxy group, a halogen atom such as a chlorine atom, or a phenyl group substituted with a substituent such as a hydroxyl group. The same applies to the aryl moiety in other substituents having an aryl moiety (such as an aryloxy group).

The C₁-C₅ alkyl group in the definition of R¹, R², and R³ may be linear or branched. Examples thereof include a methyl group, an ethyl group, a propyl group, an isopropyl group, a butyl group, an isobutyl group, a sec-butyl group, a tert-butyl group, and a pentyl group. The same applies to the alkyl moiety in other substituents having an alkyl moiety (such as an alkoxycarbonylalkyl group).

Examples of the alkoxycarbonylalkyl group having a total carbon number of 3 to 6 in the definition of R¹ include a methoxycarbonylmethyl group, an ethoxycarbonylmethyl group, a propoxycarbonylmethyl group, a methoxycarbonylethyl group, and a methoxycarbonylpropyl group.

Examples of the C₃-C₅ alkylene group in the definition of R¹ and R² include a trimethylene group, a tetramethylene group, a pentamethylene group, a methyltrimethylene group, an ethyltrimethylene group, a dimethyltrimethylene group, and a methyltetramethylene group.

Examples of the aryloxy group in the definition of R² include a p-methylphenoxy group, a p-methoxyphenoxy group, a p-chlorophenoxy group, and a p-hydroxyphenoxy group, and examples of the arylmercapto group in the definition of R² include a phenylmercapto group, a p-methylphenylmercapto group, a p-methoxyphenylmercapto group, a p-chlorophenylmercapto group, and a p-hydroxyphenylmercapto group.

Examples of the C₁-C₃ hydroxyalkyl group in the definition of R² and R³ include a hydroxymethyl group, a 2-hydroxyethyl group, and a 3-hydroxypropyl group. Examples of the C₅-C₇ cycloalkyl group in the definition of R³ include a cyclopentyl group, a cyclohexyl group, and a cycloheptyl group.

In the definition of R³, examples of the C₁-C₅ alkoxy group in a substituent of a phenyl group include a methoxy group, an ethoxy group, propoxy group, an isopropoxy group, a butoxy group, and a pentyloxy group, examples of the alkoxycarbonyl group having a total carbon number of 2 to 5 include a methoxycarbonyl group, an ethoxycarbonyl group, a propoxycarbonyl group, and a butoxycarbonyl group, examples of the C₁-C₃ alkylmercapto group include a methylmercapto group, an ethylmercapto group, and a propylmercapto group, examples of the C₁-C₄ alkylamino group include a methylamino group, an ethylamino group, a propylamino group, and a butylamino group, and examples of the dialkylamino group having a total carbon number of 2 to 8 include a dimethylamino group, a diethylamino group, a dipropylamino group, and a dibutylamino group.

Examples of the compound (I) suitably used as the active ingredient of the medicament of the present invention include the following compounds:
3-methyl-1-phenyl-2-pyrazolin-5-one;
3-methyl-1-(2-methylphenyl)-2-pyrazolin-5-one;
3-methyl-1-(3-methylphenyl)-2-pyrazolin-5-one;
3-methyl-1-(4-methylphenyl)-2-pyrazolin-5-one;
3-methyl-1-(3,4-dimethylphenyl)-2-pyrazolin-5-one;
1-(4-ethylphenyl)-3-methyl-2-pyrazolin-5-one;
3-methyl-1-(4-propylphenyl)-2-pyrazolin-5-one;
1-(4-butylphenyl)-3-methyl-2-pyrazolin-5-one;
1-(3-trifluoromethylphenyl)-3-methyl-2-pyrazolin-5-one;
1-(4-trifluoromethylphenyl)-3 -methyl-2-pyrazolin-5-one;
1-(2-methoxyphenyl)-3-methyl-2-pyrazolin-5-one;
1-(3-methoxyphenyl)-3-methyl-2-pyrazolin-5-one;
1-(4-methoxyphenyl)-3-methyl-2-pyrazolin-5-one;
1-(3,4-dimethoxyphenyl)-3-methyl-2-pyrazolin-5-one;
1-(4-ethoxyphenyl)-3-methyl-2-pyrazolin-5-one;
3-methyl-1-(4-propoxyphenyl)-2-pyrazolin-5-one;
1-(4-butoxyphenyl)-3-methyl-2-pyrazolin-5-one;
1-(2-chlorophenyl)-3-methyl-2-pyrazolin-5-one;
1-(3-chlorophenyl)-3-methyl-2-pyrazolin-5-one;
1-(4-chlorophenyl)-3-methyl-2-pyrazolin-5-one;
1-(3,4-dichlorophenyl)-3-methyl-2-pyrazolin-5-one;
1-(4-bromophenyl)-3-methyl-2-pyrazolin-5-one;
1-(4-fluorophenyl)-3-methyl-2-pyrazolin-5-one;
1-(3-chloro-4-methylphenyl)-3-methyl-2-pyrazolin-5-one;
1-(3-methylmercaptophenyl)-3-methyl-2-pyrazolin-5-one;
1-(4-methylmercaptophenyl)-3-methyl-2-pyrazolin-5-one;
4-(3-methyl-5-oxo-2-pyrazolin-1-yl) benzoic acid;
1-(4-ethoxycarbonylphenyl)-3-methyl-2-pyrazolin-5-one;
1-(4-nitrophenyl)-3-methyl-2-pyrazolin-5-one;
3-ethyl-1-phenyl-2-pyrazolin-5-one;
1-phenyl-3-propyl-2-pyrazolin-5-one;
1,3-diphenyl-2-pyrazolin-5-one;
3-phenyl-1-(p-tolyl)-2-pyrazolin-5-one;
1-(4-methoxyphenyl)-3-phenyl-2-pyrazolin-5-one;
1-(4-chlorophenyl)-3-phenyl-2-pyrazolin-5-one;
3,4-dimethyl-1-phenyl-2-pyrazolin-5-one;
4-isobutyl-3-methyl-1-phenyl-2-pyrazolin-5-one;
4-(2-hydroxyethyl)-3-methyl-1-phenyl-2-pyrazolin-5-one;
3-methyl-4-phenoxy-1-phenyl-2-pyrazolin-5-one;
3-methyl-4-phenylmercapto-1-phenyl-2-pyrazolin-5-one;
3,3',4,5,6,7-hexahydro-2-phenyl-2H-indazole-3-one;
3-(ethoxycarbonylmethyl)-1-phenyl-2-pyrazolin-5-one;
1-phenyl-2-pyrazolin-5-one;
3-methyl-2-pyrazolin-5-one;
1,3-dimethyl-2-pyrazolin-5-one;
1-ethyl-3-methyl-2-pyrazolin-5-one;
1-butyl-3-methyl-2-pyrazolin-5-one;
1-(2-hydroxyethyl)-3-methyl-2-pyrazolin-5-one;
1-cyclohexyl-3-methyl-2-pyrazolin-5-one;
1-benzyl-3-methyl-2-pyrazolin-5-one;
1-(α-naphthyl)-3-methyl-2-pyrazolin-5-one;
1-methyl-3-phenyl-2-pyrazolin-5-one;
3-methyl-1-(4-methylphenyl)-2-pyrazolin-5-one;
1-(4-butylphenyl)-3-methyl-2-pyrazolin-5-one;
1-(4-methoxyphenyl)-3-methyl-2-pyrazolin-5-one;
1-(4-butoxyphenyl)-3-methyl-2-pyrazolin-5-one;
1-(4-chlorophenyl)-3-methyl-2-pyrazolin-5-one;
1-(4-hydroxyphenyl)-3-methyl-2-pyrazolin-5-one;
1-(3,4-dihydroxyphenyl)-3-methyl-2-pyrazolin-5-one;
1-(2-hydroxyphenyl)-3-methyl-2-pyrazolin-5-one;
1-(3-hydroxyphenyl)-3-methyl-2-pyrazolin-5-one;
1-(4-hydroxyphenyl)-3-methyl-2-pyrazolin-5-one;
1-(3,4-hydroxyphenyl)-3-methyl-2-pyrazolin-5-one;
1-(4-hydroxyphenyl)-3-phenyl-2-pyrazolin-5-one;
1-(4-hydroxymethylphenyl)-3-methyl-2-pyrazolin-5-one;
1-(4-aminophenyl)-3-methyl-2-pyrazolin-5-one;
1-(4-methylaminophenyl)-3-methyl-2-pyrazolin-5-one;
1-(4-ethylaminophenyl)-3-methyl-2-pyrazolin-5-one;
1-(4-butylaminophenyl)-3-methyl-2-pyrazolin-5-one;
1-(4-dimethylaminophenyl)-3-methyl-2-pyrazolin-5-one;
1-(acetamidephenyl)-3-methyl-2-pyrazolin-5-one; and
1-(4-cyanophenyl)-3-methyl-2-pyrazolin-5-one

As an active ingredient of the medicament of the present invention, a physiologically acceptable salt may be used, in addition to a free-form compound represented by the formula (I). Examples of a physiologically acceptable salt include : salts with mineral acids such as hydrochloric acid, sulfuric acid, hydrobromide, and phosphoric acid; salts with organic acids such as methanesulfonic acid, p-toluenesulfonic acid, acetic acid, oxalic acid, citric acid, malic acid, and fumaric acid; salts with alkaline metals such as sodium and potassium; salts with alkaline earth metals such as magnesium; and salts with amines such as ammonia, ethanolamine, and 2-amino-2-methyl-1-propanol. In addition, the type of salt is not particularly limited as long as it is physiologically acceptable.

All of the compounds represented by the formula (I) are known compounds, and a person skilled in the art can readily synthesize them by a method described in, for example, JP 5-31523 B (1993).

The medicament of the present invention comprises a glutathione analogue. Examples of a glutathione include a glutathione (reduced form) and a glutathione (oxidized). The concentration of the glutathione analogue is desirably about the 1/10 mole concentration to equimolar concentration of the compound represented by the formula (I).

The concentration of the glutathione analogue in the medicament of the present invention is preferably 0.1 mM to 30 mM, more preferably 0.2 mM to 20 mM, and still more preferably 0.3 mM to 10 mM. The lower limit of the concentration of the glutathione analogue may be 0.1 mM, 0.172 mM, 0.2 mM, 0.3 mM, 0.4 mM, 0.5 mM, 0.6 mM, 0.7 mM, 0.83 mM, 0.86 mM, 1.03 mM, 1.72 mM, 2.07 mM, or 4.13 mM. The upper limit of the concentration of the glutathione analogue may be 30 mM, 20 mM, 10 mM, 9 mM, or 8.61 mM.

The medicament of the present invention comprises an aqueous solvent. Water is a preferable aqueous solvent.

The medicament of the present invention comprises none of sulfite, bisulfite, and pyrosulfite. Examples of sulfite described herein include sodium sulfite, potassium sulfite, and calcium sulfite. Examples of bisulfite described herein include sodium bisulfite, potassium bisulfite, and ammonium bisulfite. Examples of pyrosulfite described herein include sodium pyrosulfite and potassium pyrosulfite.

The medicament of the present invention is preferably free of vitamin C.

The medicament of the present invention is subjected to deoxygenation treatment. Examples of deoxygenation treatment include substitution treatment with an inert gas such as nitrogen gas.

The form of the medicament of the present invention is not particularly limited, and the medicament can be in various forms available to a person skilled in the art. The medicament of the present invention is preferably a medicament suitable for parenteral administration, and examples thereof include injections and infusions.

The following additives can be used for a medicinal composition suitable for injection or infusion: a dissolving agent or a solubilizing agent that can constitute an aqueous or in-use dissolving type injection such as distilled water for injection, physiological saline, or propylene glycol; an isotonic agent such as sodium chloride, D-mannitol, or glycerin; a pH adjuster such as an inorganic acid, an organic acid, an inorganic base, or an organic base; a buffer; and a preservative.

In a case in which the medicament of the present invention is an injection, favorable stabilization effects can be obtained when the liquid pH is in a range of 2.5 to 7.0. For adjusting the pH, a commonly used buffer and a pH adjuster can be used.

In the medicament of the present invention, the content of the compound represented by the formula (I) or a physiologically acceptable salt thereof, or a hydrate or solvate thereof is preferably 15 mg to 3000 mg, and the liquid volume of the medicament is preferably 10 mL to 2000 mL.

In the medicament of the present invention, one example of the content of the compound represented by the formula (I) or a physiologically acceptable salt thereof, or a hydrate or solvate thereof is 30 mg, and the liquid volume of the medicament is preferably 15 mL to 200 mL.

In the medicament of the present invention, another example of the content of the compound represented by the formula (I) or a physiologically acceptable salt thereof, or a hydrate or solvate thereof is 60 mg, and the liquid volume of the medicament is 30 mL to 200 mL.

In the medicament of the present invention, specific examples of the content of the compound represented by the formula (I) or a physiologically acceptable salt thereof, or a hydrate or solvate thereof in the liquid volume of the medicament include, but are not limited to, 30 mg/20 mL, 30 mg/100 mL, 60 mg/200 mL, 150 mg/100 mL, 300 mg/200 mL, 600 mg/400 mL, 1800 mg/1200 mL, and 2250 mg/1500 mL.

The dosage of the medicament of the present invention is not particularly limited. However, in a case in which the compound represented by the formula (I) is used as an active ingredient, the daily dosage by weight of the compound represented by formula (I) is generally 0.1 to 100 mg/kg by weight for oral administration and 0.1 to 100 mg/kg by weight for parenteral administration. The above dosage is preferably administered once a day or divided into 2 to 3 times a day. It is possible to administer the dosage by rapid intravenous infusion for about 3 minutes once, intravenous drip infusion for 30 minutes or 60 minutes once, and continuous infusion for 24 hours for 1 day to 3 days. The dosage may be adjusted according to the age, condition, and symptoms.

The administration time and the administration period of the medicament of the present invention are not particularly limited, and can be appropriately selected. For example, the medicament of the present invention may be administered prophylactically prior to the onset of a disease. In addition, after the onset of a disease, it may be administered for the purpose of treatment, improvement of symptoms, or prevention of deterioration of symptoms.

The administration route of the medicament of the present invention is not particularly limited and can be administered orally or parenterally. The administration route for parenteral administration is not particularly limited, and can be administered intravenously or intra-arterially.

Target diseases for administration of the medicament of the present invention are not particularly limited. However, examples thereof include stroke such as cerebral infarction or subarachnoid hemorrhage, and neurological diseases such as amyotrophic lateral sclerosis (ALS), Parkinson's disease and Alzheimer's disease. The medicament of the present invention is useful especially for improvement of neurological symptoms, disability in activities of daily living, and functional impairment associated with acute ischemic stroke, and slowing of progression of functional impairment due to amyotrophic lateral sclerosis (ALS).

The present invention will be described in more detail by the following Examples, but the present invention is not limited to the following Examples.

### Examples

### Example 1

A total volume of 20 mL of a mixture was prepared by separately dissolving 30 mg of edaravone and 25.4 mg of glutathione (reduced form) in water, followed by mixing. A screw-capped vial was filled with the mixture, and nitrogen gas was bubbled into the aqueous solution for degassing, thereby replacing the air in the vial with nitrogen. Then, the vial was tightly closed. Thus, a sample was obtained. When dissolving edaravone, in order to obtain a solution with a saturation solubility of 2 mg/mL, a sodium hydroxide solution was added dropwise for dissolution, and then, the pH was adjusted to 2.5 to 7.0 with hydrochloric acid. The sample was preserved at 60°C for 4 weeks and observed in terms of the edaravone concentration, appearance (coloring, insoluble foreign matter), and phenylhydrazine.
(8.61 mM edaravone + 4.13 mM glutathione) (nitrogen substitution) (liquid volume of 20 mL)

### Comparative Example 1

Observation was performed as in Example 1 except that 20 mg of sodium bisulfite was added, and the air atmosphere was maintained in the sample container. (8.61 mM edaravone + 4.13 mM glutathione + 9.61 mM sodium bisulfite) (under air atmosphere) (liquid volume of 20 mL)

### Comparative Example 2

Observation was performed as in Example 1 except that 20 mg of sodium bisulfite was added, 10 mg of L-cysteine was used instead of 25.4 mg of glutathione (reduced form), and the air atmosphere was maintained in the sample container.
(8.61 mM edaravone + 4.13 mM L-cysteine + 9.61 mM sodium bisulfite) (under air atmosphere) (liquid volume of 20 mL)

### Comparative Example 3

A sample was obtained in the same manner as in Example 1, and observation was performed as in Example 1 except that the air atmosphere was maintained in the sample container.
(8.61 mM edaravone + 4.13 mM glutathione) (under air atmosphere) (liquid volume of 20 mL)

### Example 2

A total volume of 100 mL of a mixture was prepared by separately dissolving 30 mg of edaravone and 25.4 mg of glutathione (reduced form) in water, followed by mixing. A screw-capped vial was filled with the mixture, and nitrogen gas was bubbled into the aqueous solution for degassing, thereby replacing the air in the vial with nitrogen. Then, the vial was tightly closed. Thus, a sample was obtained. Edaravone was dissolved and the sample was observed in the same manner as in Example 1.
(1.72 mM edaravone + 0.83 mM glutathione) (nitrogen substitution) (liquid volume of 100 mL)

### Comparative Example 4

Observation was performed as in Example 2 except that 20 mg of sodium bisulfite was added, and the air atmosphere was maintained in the sample container.
(1.72 mM edaravone + 0.83 mM glutathione + 1.92 mM sodium bisulfite) (under air atmosphere) (liquid volume of 100 mL)

### Comparative Example 5

Observation was performed as in Example 2 except that 20 mg of sodium bisulfite was added, 10 mg of L-cysteine was used instead of 25.4 mg of glutathione (reduced form), and the air atmosphere was maintained in the sample container.
(1.72 mM edaravone + 0.83 mM L-cysteine + 1.92 mM sodium bisulfite) (under air atmosphere) (liquid volume of 100 mL)

### Comparative Example 6

A sample was obtained in the same manner as in Example 2, and observation was performed as in Example 2 except that the air atmosphere was maintained in the sample container.
(1.72 mM edaravone + 0.83 mM glutathione) (under air atmosphere) (liquid volume of 100 mL)

### Example 3

An aqueous solution containing 8.61 mM edaravone and 4.13 mM glutathione (reduced form) was prepared. A 10-mL heat-resistant glass vial with a cap was filled with the aqueous solution, and nitrogen gas was bubbled into the aqueous solution for degassing, thereby replacing the air in the vial with nitrogen. Then, the vial was tightly closed. Thus, a sample was obtained. Edaravone was dissolved in the same manner as in Example 1. The edaravone concentration was calculated from three samples. Each sample was preserved at 60°C for 4 weeks and observed in terms of the appearance (coloring, insoluble foreign matter).
(8.61 mM edaravone + 4.13 mM glutathione) (nitrogen substitution) (liquid volume of 10 mL)

### Comparative Example 7

Observation was performed as in Example 3 except that 9.61 mM sodium bisulfite was added, and the air atmosphere was maintained in the sample container.
(8.61 mM edaravone + 4.13 mM glutathione + 9.61 mM sodium bisulfite) (under air atmosphere) (liquid volume of 10 mL)

### Comparative Example 8

A sample was obtained in the same manner as in Example 3, and observation was performed as in Example 3 except that the air atmosphere was maintained in the sample container.
(8.61 mM edaravone + 4.13 mM glutathione) (under air atmosphere) (liquid volume of 10 mL)

### Example 4

An aqueous solution of 8.61 mM edaravone and 0.86 mM glutathione (reduced form)(i.e., the 1/10 mole concentration of edaravone) was prepared. A 10-mL heat-resistant glass vial with a cap was filled with the aqueous solution, and nitrogen gas was bubbled into the aqueous solution for degassing, thereby replacing the air in the vial with nitrogen. Then, the vial was tightly closed. Thus, a sample was obtained. Edaravone was dissolved in the same manner as in Example 1. The edaravone concentration was calculated from three samples. Each sample was preserved at 60°C for 4 weeks and observed in terms of the appearance (coloring, insoluble foreign matter).
(8.61 mM edaravone + 0.86 mM glutathione) (nitrogen substitution) (liquid volume of 10 mL)

### Example 5

Observation was performed as in Example 4 except that the glutathione (reduced form) in Example 4 was changed to 1.03 mM glutathione.
(8.61 mM edaravone + 1.03 mM glutathione) (nitrogen substitution) (liquid volume of 10 mL)

### Example 6

Observation was performed as in Example 4 except that the glutathione (reduced form) in Example 4 was changed to 2.07 mM glutathione.
(8.61 mM edaravone + 2.07 mM glutathione) (nitrogen substitution) (liquid volume of 10 mL)

### Example 7

Observation was performed as in Example 4 except that the glutathione (reduced form) in Example 4 was changed to 4.13 mM glutathione.
(8.61 mM edaravone + 4.13 mM glutathione) (nitrogen substitution) (liquid volume of 10 mL)

### Example 8

Observation was performed as in Example 4 except that the glutathione (reduced form) in Example 4 was changed to 8.61 mM glutathione (i.e., the concentration equimolar to edaravone).
(8.61 mM edaravone + 8.61 mM glutathione) (nitrogen substitution) (liquid volume of 10 mL)

### Comparative Example 9

Observation was performed as in Example 4 except that 0.86 mM glutathione (reduced form) was removed from Example 4.
(8.61 mM edaravone) (nitrogen substitution) (liquid volume of 10 mL)

### Test Example 1

### (a) Edaravone concentration

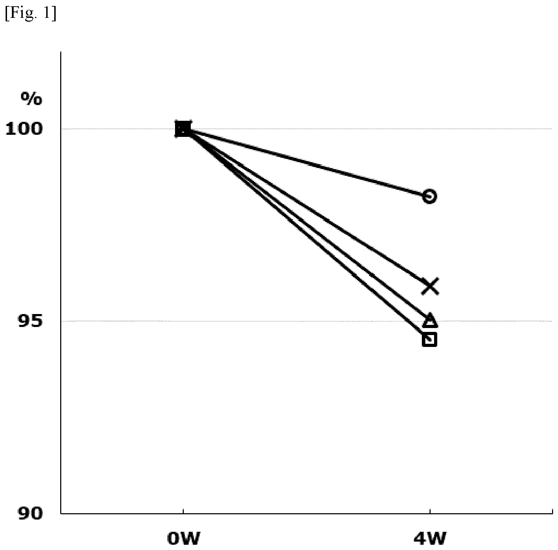

Fig. 1 shows the edaravone concentrations of the samples obtained in Example 1 and Comparative Examples 1, 2, and 3 (60°C, 4 weeks later, liquid volume of 20 mL). Notes to Fig. 1:
○: Example 1 (edaravone + glutathione) (nitrogen substitution)
×: Comparative Example 1 (edaravone + glutathione + sodium bisulfite) (under air atmosphere)
Δ: Comparative Example 2 (edaravone + cysteine + sodium bisulfite) (under air atmosphere)
□: Comparative Example 3 (edaravone + glutathione) (under air atmosphere)

The edaravone concentration was measured based on the ratio of the peak area after 4 weeks to the peak area value of a 8.61 mM edaravone standard sample by high performance liquid chromatography (HPLC).

HPLC measurement method and conditions:
Detector: Ultraviolet absorptiometer (measurement wavelength: 295 nm)
Column: CAPCELL PAK ADME (Shiseido Company, Limited), 5µm, 4.6 mm I.D. × 250 mm
Mobile phase: 40 mM NaH₂PO₄: methanol = 40:60,v/v
Flow rate: 0.5 mL/min

### (b) Appearance

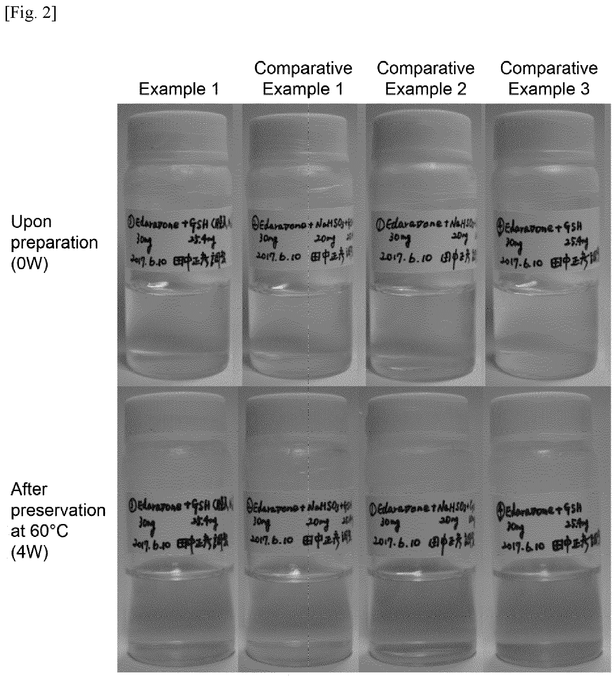

Fig. 2 is a view of pictures of the samples showing the appearance of each sample (liquid volume of 20 mL).

[Table 1]

**Table 1. Coloring (60°C, 4 weeks later, liquid volume of 20 mL)**

| Sample | Degree of coloring |
|---|---|
| Example 1 (edaravone + glutathione) (nitrogen substitution) | - |
| Comparative Example 1 (edaravone + glutathione + sodium bisulfite) (under air atmosphere) | - |
| Comparative Example 2 (edaravone + cysteine + sodium bisulfite) (under air atmosphere) | ± |
| Comparative Example 3 (edaravone + glutathione) (under air atmosphere) | ± |

| | |
|---|---|
| - Colorless and clear ± Very slight coloring + Slight coloring ++ Light coloring +++ Obvious coloring | |

[Table 2]

**Table 2. Insoluble foreign matter (60°C, 4 weeks later, liquid volume of 20 mL)**

| Sample | Degree of insoluble foreign matter |
|---|---|
| Example 1 (edaravone + glutathione) (nitrogen substitution) | - |
| Comparative Example 1 (edaravone + glutathione + sodium bisulfite) (under air atmosphere) | - |
| Comparative Example 2 (edaravone + cysteine + sodium bisulfite) (under air atmosphere) | ± |
| Comparative Example 3 (edaravone + glutathione) (under air atmosphere) | + |

| | |
|---|---|
| - No insoluble foreign matter ± Very little insoluble foreign matter + Little fine insoluble foreign matter ++ Fine insoluble foreign matter +++ Large mass or crystalline insoluble foreign matter | |

### (c) Phenylhydrazine (PHZ)

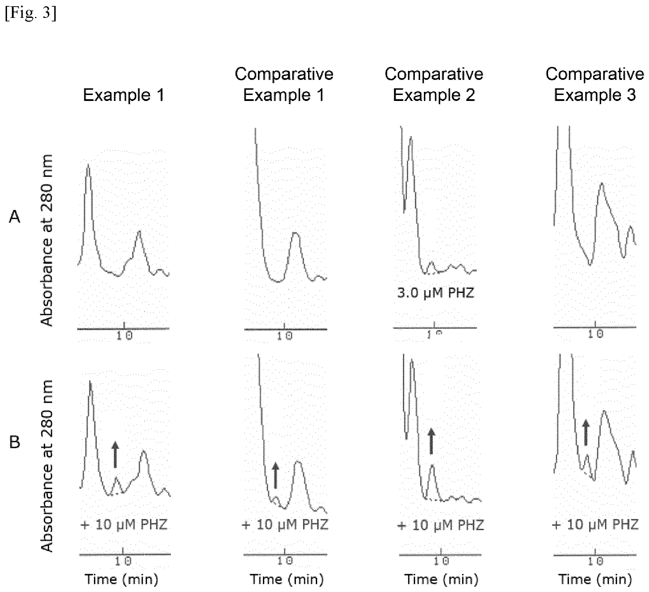

The presence or absence of phenylhydrazine was examined by HPLC. With the use of 1, 10, and 100 µM phenylhydrazine hydrochloride standard samples, the presence or absence of a peak corresponding to the retention time of each thereof was observed. In addition, equal liquid volumes of each sample and 10 µM phenylhydrazine were mixed (spike). Thus, the peak of the spike was confirmed to correspond to phenylhydrazine (Fig. 3).

Notes to Fig. 3:
Example 1 (edaravone + glutathione) (nitrogen substitution)
Comparative Example 1 (edaravone + glutathione + sodium bisulfite) (under air atmosphere)
Comparative Example 2 (edaravone + cysteine + sodium bisulfite) (under air atmosphere)
Comparative Example 3 (edaravone + glutathione) (under air atmosphere)
   A: Before phenylhydrazine spike
   B: After phenylhydrazine (10 µM) spike

HPLC measurement method and conditions:
Detector: Ultraviolet absorptiometer (measurement wavelength: 280nm)
Column: CAPCELL PAK ADME (Shiseido Company, Limited), 5 µm, 4.6 mm I.D. × 250 mm
Mobile phase: 40 mM NaH₂PO₄: methanol = 40:60,v/v
Flow rate: 0.5 mL/min

[Table 3]

**Table 3. Phenyl hydrazine (60°C, 4 weeks later, liquid volume of 20 mL)**

| Sample | Presence or absence of phenylhydrazine |
|---|---|
| Example 1 (edaravone + glutathione) (nitrogen substitution) | - |
| Comparative Example 1 (edaravone + glutathione + sodium bisulfite) (under air atmosphere) | - |
| Comparative Example 2 (edaravone + cysteine + sodium bisulfite) (under air atmosphere) | + (3.0 µM) |
| Comparative Example 3 (edaravone + glutathione) (under air atmosphere) | - |

### Test Example 2

### (a) Edaravone concentration

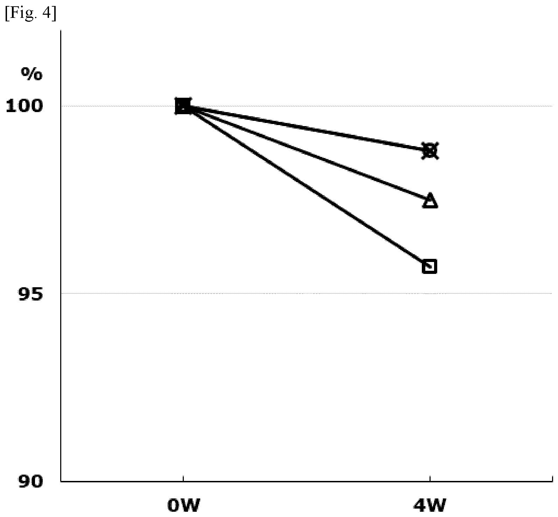

Fig. 4 shows the edaravone concentrations of the samples obtained in Example 2 and Comparative Examples 4, 5, and 6 (60°C, 4 weeks later, liquid volume of 100 mL). The edaravone concentration was measured using a 1.72 mM edaravone standard sample by the same method as in Test Example 1.

Notes to Fig. 4:
○: Example 2 (edaravone + glutathione) (nitrogen substitution)
×: Comparative Example 4 (edaravone + glutathione + sodium bisulfite) (under air atmosphere)
Δ: Comparative Example 5 (edaravone + cysteine + sodium bisulfite) (under air atmosphere)
□: Comparative Example 6 (edaravone + glutathione) (under air atmosphere)

### (b) Appearance

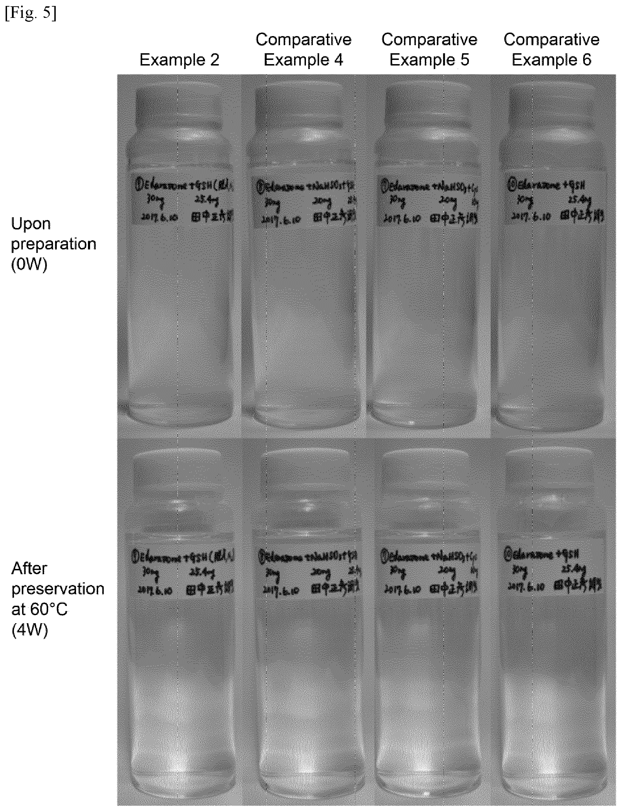

Fig. 5 is a view of pictures of the samples showing the appearance of each sample (liquid volume of 100 mL).

[Table 4]

**Table 4. Coloring (60°C, 4 weeks later, liquid volume of 100 mL)**

| Sample | Degree of coloring |
|---|---|
| Example 2 (edaravone + glutathione) (nitrogen substitution) | - |
| Comparative Example 4 (edaravone + glutathione + sodium bisulfite) (under air atmosphere) | - |
| Comparative Example 5 (edaravone + cysteine + sodium bisulfite) (under air atmosphere) | - |
| Comparative Example 6 (edaravone + glutathione) (under air atmosphere) | ± |

| | |
|---|---|
| - Colorless and clear ± Very slight coloring + Slight coloring ++ Light coloring +++ Obvious coloring | |

[Table 5]

**Table 5. Insoluble foreign matter (60°C, 4 weeks later, liquid volume of 100 mL)**

| Sample | Degree of insoluble foreign matter |
|---|---|
| Example 2 (edaravone + glutathione) (nitrogen substitution) | - |
| Comparative Example 4 (edaravone + glutathione + sodium bisulfite) (under air atmosphere) | - |
| Comparative Example 5 (edaravone + cysteine + sodium bisulfite) (under air atmosphere) | ± |
| Comparative Example 6 (edaravone + glutathione) (under air atmosphere) | ± |

| | |
|---|---|
| - No insoluble foreign matter ± Very little insoluble foreign matter + Little fine insoluble foreign matter ++ Fine insoluble foreign matter +++ Large mass or crystalline insoluble foreign matter | |

### (c) Phenylhydrazine

The presence or absence of phenylhydrazine was examined by HPLC.

[Table 6]

**Table 6. Phenyl hydrazine (60°C, 4 weeks later, liquid volume of 100 mL)**

| Sample | Presence or absence of phenylhydrazine |
|---|---|
| Example 2 (edaravone + glutathione) (nitrogen substitution) | - |
| Comparative Example 4 (edaravone + glutathione + sodium bisulfite) (under air atmosphere) | - |
| Comparative Example 5 (edaravone + cysteine + sodium bisulfite) (under air atmosphere) | - |
| Comparative Example 6 (edaravone + glutathione) (under air atmosphere) | - |

### Test Example 3

### (a) Edaravone concentration

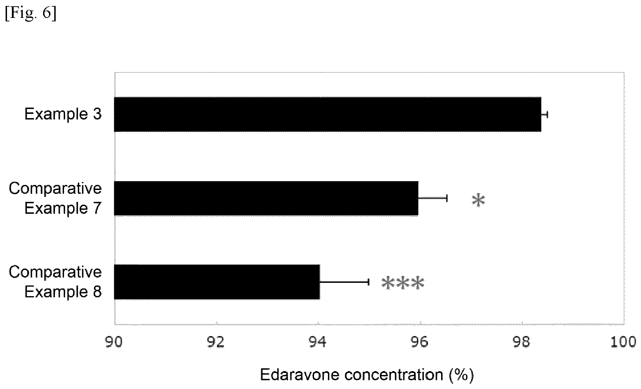

Fig. 6 shows the edaravone concentrations of the samples obtained in Example 3 and Comparative Examples 7 and 8 (60°C, 4 weeks later, 10 mL). The edaravone concentration was measured by the same method as in Test Example 1.

Notes to Fig. 6:
ANOVA: p = 0.0006
Scheffe's multiple comparison:
   * p = 0.0122 (Example 3 vs Comparative Example 7)
   *** p = 0.0006 (Example 3 vs Comparative Example 8)

### (b) Appearance

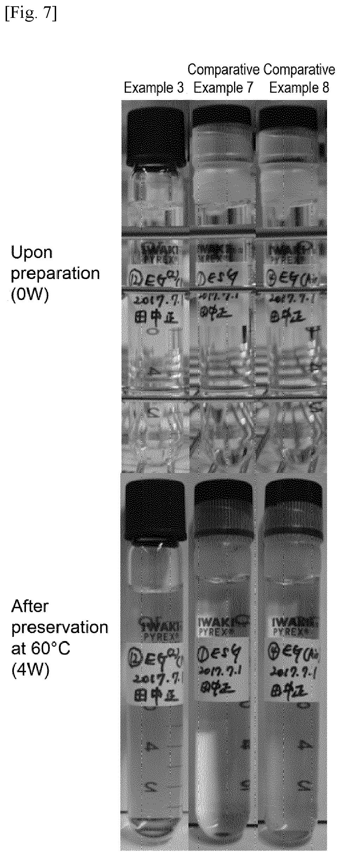

Fig. 7 is a view of pictures of the samples showing the appearance of each sample (liquid volume of 100 mL).

[Table 7]

**Table 7. Coloring (60°C, 4 weeks later, liquid volume of 10 mL)**

| Sample | Degree of coloring |
|---|---|
| Example 3 (edaravone + glutathione) (nitrogen substitution) | - |
| Comparative Example 7 (edaravone + glutathione + sodium bisulfite) (under air atmosphere) | - |
| Comparative Example 8 (edaravone + glutathione) (under air atmosphere) | ± |

| | |
|---|---|
| - Colorless and clear ± Very slight coloring + Slight coloring ++ Light coloring +++ Obvious coloring | |

[Table 8]

**Table 8. Insoluble foreign matter (60°C, 4 weeks later, liquid volume of 10 mL)**

| Sample | Degree of insoluble foreign matter |
|---|---|
| Example 3 (edaravone + glutathione) (nitrogen substitution) | - |
| Comparative Example 7 (edaravone + glutathione + sodium bisulfite) (under air atmosphere) | - |
| Comparative Example 8 (edaravone + glutathione) (under air atmosphere) | + |

| | |
|---|---|
| - No insoluble foreign matter ± Very little insoluble foreign matter + Little fine insoluble foreign matter ++ Fine insoluble foreign matter +++ Large mass or crystalline insoluble foreign matter | |

### Test Example 4

### (a) Appearance

[Table 9]

**Table 9. Coloring (60°C, 4 weeks later, liquid volume of 10 mL)**

| Sample | Degree of coloring |
|---|---|
| Example 4 (8.61 mM edaravone + 0.86 mM glutathione) (nitrogen substitution) | - |
| Example 5 (8.61 mM edaravone + 1.03 mM glutathione) (nitrogen substitution) | - |
| Example 6 (8.61 mM edaravone + 2.07 mM glutathione) (nitrogen substitution) | - |
| Example 7 (8.61 mM edaravone + 4.13 mM glutathione) (nitrogen substitution) | - |
| Example 8 (8.61 mM edaravone + 8.61 mM glutathione) (nitrogen substitution) | - |
| Comparative Example 9 (8.61 mM edaravone) (nitrogen substitution) | - |

| | |
|---|---|
| - Colorless and clear ± Very slight coloring + Slight coloring ++ Light coloring +++ Obvious coloring | |

[Table 10]

**Table 10. Insoluble foreign matter (60°C, 4 weeks later, liquid volume of 10 mL)**

| Sample | Degree of insoluble foreign matter |
|---|---|
| Example 4 (8.61 mM edaravone + 0.86 mM glutathione) (nitrogen substitution) | - |
| Example 5 (8.61 mM edaravone + 1.03 mM glutathione) (nitrogen substitution) | - |
| Example 6 (8.61 mM edaravone + 2.07 mM glutathione) (nitrogen substitution) | - |
| Example 7 (8.61 mM edaravone + 4.13 mM glutathione) (nitrogen substitution) | - |
| Example 8 (8.61 mM edaravone + 8.61 mM glutathione) (nitrogen substitution) | - |
| Comparative Example 9 (8.61 mM edaravone) (nitrogen substitution) | + |

| | |
|---|---|
| - No insoluble foreign matter ± Very little insoluble foreign matter + Little fine insoluble foreign matter ++ Fine insoluble foreign matter +++ Large mass or crystalline insoluble foreign matter | |

### (b) Edaravone concentration

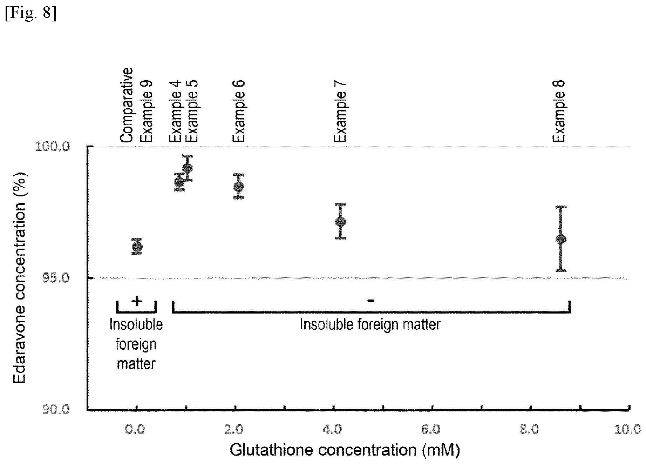

Fig. 8 shows the edaravone concentrations of the samples obtained in Examples 4, 5, 6, 7, and 8 and Comparative Example 9 (60°C, 4 weeks later, 10 mL). The edaravone concentration was measured by the same method as in Test Example 1. In addition, the presence or absence of insoluble foreign matter was indicated.

## Claims

1. A medicament, comprising:
(a) a compound represented by the following formula (I) or a physiologically acceptable salt thereof, or a hydrate or solvate thereof;
(b) a glutathione analogue, and
(c) an aqueous solvent,
which comprises none of sulfite, bisulfite, and pyrosulfite, and is subjected to deoxygenation treatment: 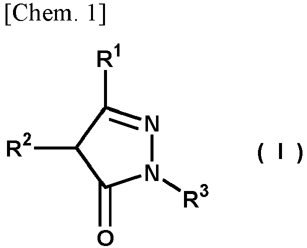 wherein R¹ represents a hydrogen atom, aryl, C₁-C₅ alkyl, or alkoxycarbonylalkyl having a total carbon number of 3 to 6, and R² represents a hydrogen atom, aryloxy, arylmercapto, C₁-C₅ alkyl, or C₁-C₃ hydroxyalkyl; or R¹ and R² together represent C₃-C₅ alkylene, and R³ represents a hydrogen atom, C₁-C₅ alkyl, C₅-C₇ cycloalkyl, C₁-C₃ hydroxyalkyl, benzyl, naphthyl, or phenyl, or phenyl substituted with 1 to 3 identical or different substituents selected from the group consisting of C₁-C₅ alkoxy, C₁-C₃ hydroxyalkyl, alkoxycarbonyl having a total carbon number of 2 to 5, C₁-C₃ alkylmercapto, C₁-C₄ alkylamino, dialkylamino having a total carbon number of 2 to 8, a halogen atom, trifluoromethyl, carboxyl, cyano, a hydroxy group, nitro, amino, and acetamide.

2. The medicament according to claim 1, wherein the compound represented by the formula (1) is 3-methyl-1-phenyl-2-pyrazolin-5-one.

3. The medicament according to claim 1 or 2 wherein the glutathione analogue is glutathione.

4. The medicament according to any one of claims 1 to 3, wherein the aqueous solvent is water.

5. The medicament according to any one of claims 1 to 4, wherein the deoxygenation treatment is nitrogen substitution treatment.

6. The medicament according to any one of claims 1 to 5, which is an injection.

7. The medicament according to any one of claims 1 to 6, wherein the content of the compound represented by the formula (I) or a physiologically acceptable salt thereof, or a hydrate or solvate thereof is 15 mg to 3000 mg, and the liquid volume of the medicament is 10 mL to 2000 mL.

8. The medicament according to any one of claims 1 to 7, wherein the content of the compound represented by the formula (I) or a physiologically acceptable salt thereof, or a hydrate or solvate thereof is 30 mg, and the liquid volume of the medicament is 15 mL to 200 mL.

9. The medicament according to any one of claims 1 to 7, wherein the content of the compound represented by the formula (I) or a physiologically acceptable salt thereof, or a hydrate or solvate thereof is 60 mg, and the liquid volume of the medicament is 30 mL to 200 mL.

10. The medicament according to any one of claims 1 to 7, wherein the content of the compound represented by the formula (I) or a physiologically acceptable salt thereof, or a hydrate or solvate thereof in the liquid volume of the medicament is 30 mg/20 mL, 30 mg/100 mL, 60 mg/200 mL, 150 mg/100 mL, 300 mg/200 mL, 600 mg/400 mL, 1800 mg/1200 mL, or 2250 mg/1500 mL.
